# EUROPÄISCHE PATENTSCHRIFT

(11) **EP 1 519 724 B1**
(45) Veröffentlichungstag und Bekanntmachung des Hinweises auf die Patenterteilung: **09.09.2009**
(21) Anmeldenummer: 03762678.5
(22) Anmeldetag: 09.07.2003
(51) Int. Cl.: A61K 31/438, C07D 221/20, A61P 37/06, A61P 35/00, A61P 33/00

(54) **FREDERICAMYCIN-DERIVATE ALS ARZNEIMITTEL ZUR TUMORBEHANDLUNG**
FREDERICAMYCIN DERIVATIVES AS MEDICAMENTS FOR TREATING TUMOURS
DERIVES DE FREDERICAMYCINE UTILISES COMME MEDICAMENTS POUR LE TRAITEMENT DE TUMEURS

(30) Priorität: 09.07.2002 DE 10230917
(43) Veröffentlichungstag der Anmeldung: 06.04.2005
(73) Patentinhaber: Zentopharm GmbH, 22525 Hamburg (DE)
(72) Erfinder: SIMON, Werner, 55595 Hüffelsheim (DE); ABEL, Ulrich, 69126 Heidelberg (DE)
(74) Vertreter: Simandi, Claus
(86) Internationale Anmeldenummer: PCT/EP2003/007427
(87) Internationale Veröffentlichungsnummer: WO 2004/004713

(56) Entgegenhaltungen:
- US-A- 4 584 377
- US-A- 4 673 678
- US-A- 5 166 208
- WARNICK-PICKLE D J ET AL: "FREDERICAMYCIN A. A NEW ANTITUMOR ANTIBIOTIC BIOLOGICAL PROPERTIES" JOURNAL OF ANTIBIOTICS, JAPAN ANTIBIOTICS RESEARCH ASSOCIATION. TOKYO, JP, Bd. 34, Nr. 11, 1981, Seiten 1402-1407, XP000915616 ISSN: 0021-8820
- LATHAM M D ET AL: "INHIBITION OF TOPOISOMERASES BY FREDERICAMYCIN A" CANCER CHEMOTHERAPY AND PHARMACOLOGY, SPRINGER VERLAG, BERLIN, DE, Bd. 24, Nr. 3, 1989, Seiten 167-171, XP001087670 ISSN: 0344-5704

## Beschreibung

Die Erfindung betrifft neue Fredericamycin-Derivate, Arzneimittel die diese oder deren Salze enthalten, und die Verwendung der Fredericamycin-Derivate zur Behandlung von Erkrankungen, insbesondere Tumorerkrankungen.

Fredericamycin wurde 1981 aus Streptomyces griseus isoliert und zeigt Antitumoraktivität.

Fredericamycin und einige Fredericamycin-Derivate sind bekannt.

In Heterocycles 37 (1994) 1893 - 1912, J. Am. Chem. Soc. 116 (1994) 9921 - 9926, J. Am. Chem. Soc. 116 (1994) 11275 -11286, J. Am. Chem. Soc. 117 (1995) 11839 - 11849 und in J. Am. Chem. Soc. 123 (2001) sind verschiedene, auch enantioselektive, Totalsynthesen von Fredericamycin A beschrieben.

In US 4673768 sind Alkalisalze des Fredericamycin A beschrieben. In US 4584377 Fredericamycin-Derivate, insbesondere am Ring E und F acylierte Derivate, beschrieben. In US 5,166,208 sind ebenso Fredericamycin-Derivate beschrieben, insbesondere Derivate, die am Ring F Thio- oder Amino-substituenten tragen. Die Derivate werden semisynthetisch oder totalsynthetisch hergestellt.

Überraschenderweise wurde gefunden, dass Fredericamycin-Derivate, die insbesondere am Ring E, am Ring F oder an den Ringen E und F derivatisiert sind, potente Arzneimittel darstellen. Es wurde außerdem eine semisynthetische Möglichkeit gefunden Reste am Ring E, am Ring F oder an beiden Ringen E und F einzuführen, die erlauben unter anderem die Wasserlöslichkeit der Derivate zu erhöhen. Weitere aus dem Stand der Technik bekannte Wege zur Derivatisierung können an den erfindungsgemäßen Derivaten zusätzlich durchgeführt werden. Es wurde des weiteren eine Alternative gefunden Fredericamycin-Derivate wasserlöslich zu machen, in dem Cyclodextrin Einschlussverbindungen hergestellt werden.

Die Erfindung betrifft neue Fredericamycin-Derivate der allgemeinen Formel Ia oder Ib: 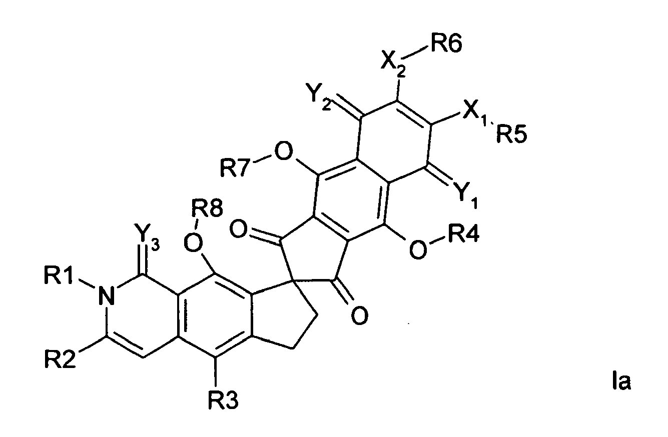 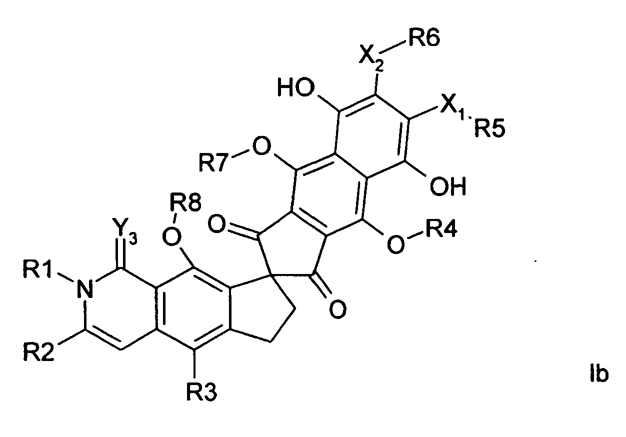 wobei jeweils
- R1: H, C₁-C₆-Alkyl, Cycloalkyl, C₁-C₄-Alkyl-Cycloalkyl,
- R2: C₁-C₁₄-Alkyl, C₂-C₁₄-Alkenyl, C₁-C₄-Alkyl-Aryl, Heteroaryl, C₁-C₄-Alkyl-Heteroaryl, Cycloalkyl, C₁-C₄- Alkyl-Cycloalkyl, Heterocycloalkyl, C₁-C₄-Alkyl- Heterocycloalkyl, CₘH₂ₘ₊ₒ₋ₚYₚ (mit m = 1 bis 6, für o = 1, p = 1 bis 2m+o; für m = 2 bis 6, o = -1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), CH₂NHCOR21, CH₂NHCSR21, CH₂S(O)nR21 mit n=0,1,2, CH₂SCOR21, CH₂OSO₂-R21, CHO, CH=NOH, CH(OH)R21, -CH=NOR21, -CH=NOCOR21, -CH=NOCH₂CONR21R22, -CH=NOCH(CH₃)CONR21R22, -CH=NOC(CH₃)₂CONR21R22, -CH=N-NHCO-R23, -CH=N-NHCO- CH₂NHCOR21, -CH=N-O-CH₂NHCOR21, -CH=N-NHCS-R23, -CH=CR24R25 (trans oder cis), COOH, COOR21, CONR21R22, -CH=NR21, -CH=N-NR21R22, 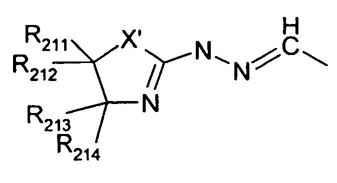 (mit X' = NR215, O, S und R211, R212, R213, R214, R215 unabhängig voneinander H oder C₁-C₆-Alkyl), -CH=N-NHSO₂-Aryl, -CH=N-NHSO₂-Heteroaryl,
- R21, R22: unabhängig voneinander C₁-C₁₄-Alkyl, C₁-C₁₄-Alkanoyl, C₁-C₆- Alkylhydroxy, C₁-C₆-Alkylamino, C₁-C₆-Alkylamino-C₁-C₆- Alkyl, C₁-C₆-Alkylamino-di-C₁-C₆-Alkyl, Cycloalkyl, C₁-C₄- Alkyl-Cycloalkyl, Heterocycloalkyl, C₁-C₄-Alkyl- Heterocycloalkyl, Aryl, Aryloyl, C₁-C₄-Alkyl-Aryl, Heteroaryl, Heteroaryloyl, C₁-C₄-Alkyl-Heteroaryl, Cycloalkanoyl, C₁-C₄-Alkanoyl-Cycloalkyl, Heterocycloalkanoyl, C₁-C₄-Alkanoyl-Heterocycloalkyl, C₁- C₄-Alkanoyl-Aryl, C₁-C₄-Alkanoyl-Heteroaryl, Mono- und Di- Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschließlich ihren Desoxyverbidungen (wie z.B. Glucose, Desoxyglucose, Ribose, Desoxyribose),
- R23: unabhängig von R21, die selben Bedeutungen wie R21 oder CH₂pyridinium-salze, CH2tri-C₁-C₆-alkylammonmium-salze,
- R24: unabhängig von R21, die selben Bedeutungen wie R21 oder H, CN, COCH₃, COOH, COOR21, CONR21R22, NH₂, NHCOR21
- R25: unabhängig von R21, die selben Bedeutungen wie R21 oder H, CN, COCH₃, COOH, COOR21, CONR21R22, NH₂, NHCOR21
- R24,R25: zusammen C₄-C₈-Cycloalkyl,
- R3: H, F, Cl, Br, I, , OH, OR31, NO₂, NH₂, NHR31, NR31R32, NHCHO, NHCOR31, NHCOCF₃, CH₃₋ₘHalₘ (mit Hal = Cl, F, insbesondere F, und m = 1, 2, 3), OCOR31,
- R31,R32: unabhängig voneinander C₁-C₆-Alkyl,
- R5,R6: unabhängig voneinander H, C₁-C₁₄-Alkyl, C₂-C₁₄-Alkenyl, Aryl, C₁-C₄-Alkyl-Aryl, Heteroaryl, C₁-C₄-Alkyl-Heteroaryl, Cycloalkyl, C₁-C₄-Alkyl-Cycloalkyl, Heterocycloalkyl, C₁- C₄-Alkyl-Heterocycloalkyl, CₘH₂ₘ₊ₒ₋ₚYₚ (mit m = 1 bis 6, für o = 1, p = 1 bis 2m+o; für m = 2 bis 6, o = -1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), oder R5 und R6 ergeben zusammen mit X₁-C-C-X₂ einen 5,6 oder 7 gliedrigen Ring,
- R4,R7,R8: unabhängig voneinander H, C₁-C₆-Alkyl, CO-R41
- R41: unabhängig von R21, die selben Bedeutungen wie R21
- X1: O, S, NH, N-C₁-C₈-Alkyl, N-Cycloalkyl
- X2: O, S, NH, N-C₁-C₈-Alkyl, N-Cycloalkyl
- Y1: O, N-R9, wobei R9 unabhängig von R5 die selben Bedeutungen wie R5 annehmen kann,
- Y2: O, N-R10, wobei R10 unabhängig von R5 die selben Bedeutungen wie R5 annehmen kann, und bei Y1 oder Y2 gleich N-R9 oder N-R10, kann X2-R6 gleich H sein,
- Y3: 0, S, NH,
bedeutet, deren Stereoisomere, Tautomere und deren physiologisch verträglichen Salze oder Einschlussverbindungen.

Bevorzugt sind Verbindungen der Formel IIa oder IIb 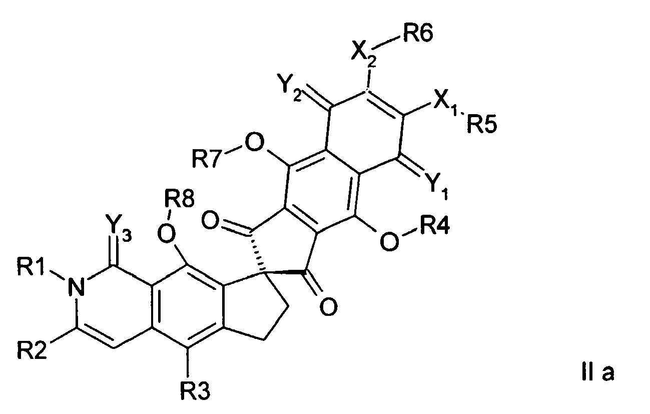 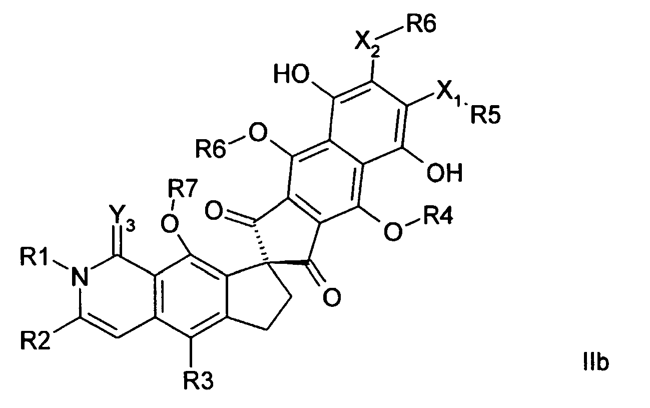 wobei die Bedeutung der Reste R1-R41, X1, X2, Y1 und Y2 wie oben angegeben ist, deren Tautomere und deren physiologisch verträglichen Salze oder Einschlussverbindungen.

Die Erfindung betrifft außerdem Verbindungen der Formel Ia, Ib, IIa oder IIb, bei denen die Reste R, die oben angegebenen Bedeutungen haben und R2 gegenüber R2 gleich CH=CH-CH=CH-CH₃ die Wasserlöslichkeit bei Beibehaltung aller anderer Reste mindestens verdoppelt, bevorzugt mindestens verfünffacht, mehr bevorzugt mindestens verzehnfacht, besonders bevorzugt mindestens verfünfzigfacht, insbesondere verhundertfacht, oder sogar verfünfhundertfacht. Die Erhöhung der Wasserlöslichkeit geschieht z.B. über die Einführung von Gruppen, die vermehrt Wasserstoffbrückenbindungen ausbilden können und/oder polar und/oder ionisch sind. Ein Schlüsselzwischenprodukt sind Verbindungen mit einer Aldehyd Funktion in R2. Bevorzugt sind bei den Aldehyden und den abgeleiteten Verbindungen, solche bei denen zumindest R1 oder R3 ungleich H ist, wenn R4 bis R8 H oder Alkyl sind.

Bevorzugte Reste bei R2 sind Heteroaryl, Cycloalkyl, C₁-C₄-Alkyl-Cycloalkyl, Heterocycloalkyl, C₁-C₄-Alkyl-Heterocycloalkyl, CₘH₂ₘ₊ₒ₋ₚYₚ (mit m = 1 bis 6, für o = 1, p = 1 bis 2m+o; für m = 2 bis 6, o =-1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), CH₂NHCOR21, CH₂NHCSR21, CH₂S(O)nR21 mit n=0,1,2, CH₂SCOR21, CH₂OSO₂-R21, CH(OH)R21, -CH=NOCOR21, -CH=NOCH₂CONR21R22, -CH=NOCH(CH₃)CONR21R22, -CH=NOC(CH₃)₂CONR21R22, -CH=N-NHCO-R23, -CH=N-NHCO-CH₂NHCOR21, -CH=N-O-CH₂NHCOR21, -CH=N-NHCS-R23, -CH=CR24R25 ( trans oder cis ), CONR21R22, -CH=NR21, -CH≡N-NR21R22, 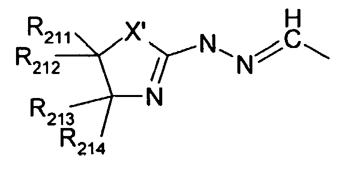 (mit X' = NR215, O, S und R211, R212, R213, R214, R215 unabhängig voneinander H oder C₁-C₆-Alkyl), -CH=N-NHSO₂-Aryl, -CH=N-NHSO₂-Heteroaryl,

Bevorzugt sind weiterhin Verbindungen wie oben angegeben, wobei die Reste R bevorzugt unabhängig voneinander eine oder mehrere der folgenden Bedeutungen annehmen:
- R1: H, C₁-C₅-Alkyl, Cycloalkyl, insbesondere H,
- R2: C₁-C₅-Alkyl, C₁-C₄-Alkyl-Aryl, C₂-C₅-Alkenyl, Heteroaryl, C₁-C₄-Alkyl-Heteroaryl, CHF₂, CF₃, Polyolseitenkette insbesondere CHOH-CHOH-CHOH-CHOH-CH₃, CHOH-CHOH-CH=CH-CH₃, CH=CH-CHOH-CHOH-CH₃, CH₂Y (Y=F,Cl,Br,I) , ), CH₂NH₂, CH₂NR21R22, CH₂NHCOR23, CH₂NHCSR23, CH₂SH, CH₂S(O)nR21 mit n=0,1,2, CH₂SCOR21, insbesondere CH₂OH, CH₂OR21, CH₂OSO₂-R21, insbesondere CHO, CH(OR21)₂, CH(SR21)₂, CN, CH=NOH, CH=NOR21, CH=NOCOR21, CH=N-NHCO-R23, CH=CR24,R25 ( trans oder cis ), insbesondere COOH (insbesondere deren physiologisch verträglichen Salze), COOR21, CONR21R22, -CH=NR21, -CH=N-NR21R22, 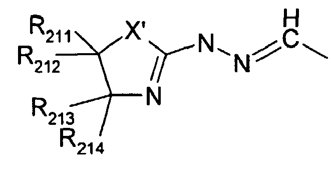 (mit X' = NR215, O, S und R211, R212, R213, R214, R215 unabhängig voneinander H oder C₁-C₆-Alkyl) , -CH=N-NHSO₂-Aryl, -CH=N-NHSO₂-Heteroaryl, CH=N-NHCO-R23,
- R21, R22: unabhängig voneinander C₁-C₆-Alkyl, Cycloalkyl, Aryl, C₁- C₄-Alkyl-Aryl, Heteroaryl, C₁-C₄-Alkyl-Heteroaryl
- R23: unabhängig von R21, die selben Bedeutungen wie R21 oder CH₂pyridinium-salze, CH2tri-C₁-C₆-alkylammonmium-salze,
- R24: unabhängig von R21, die selben Bedeutungen wie R21 oder H, CN, COCH₃, COOH, COOR21, CONR21R22, NH₂, NHCOR21
- R25: unabhängig von R21, die selben Bedeutungen wie R21 oder H, CN, COCH₃, COOH, COOR21, CONR21R22, NH₂, NHCOR21
- R24,R25: zusammen C₄-C₈-Cycloalkyl,
- R3: F, Cl, Br, I, NO₂, NH₂, NHCOR31,
- R31: unabhängig voneinander C₁-C₆-Alkyl,
- R5,R6: H, unabhängig voneinander C₁-C₁₄-Alkyl, C₂-C₁₄-Alkenyl, Aryl, C₁-C₄-Alkyl-Aryl, Heteroaryl, C₁-C₄-Alkyl-Heteroaryl, Cycloalkyl, C₁-C₄-Alkyl-Cycloalkyl, Heterocycloalkyl, C₁- C₄-Alkyl-Heterocycloalkyl, CₘH₂ₘ₊ₒ₋ₚYₚ (mit m = 1 bis 6, für o = 1, p = 1 bis 2m+o; für m = 2 bis 6, o = -1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), oder R5 und R6 ergeben zusammen mit X₁-C-C-X₂ einen 5,6 oder 7 gliedrigen Ring,
- R4,R7,R8: unabhängig voneinander H, C₁-C₅-Alkyl, CO-R41
- R41: unabhängig von R21, die selben Bedeutungen wie R21
- Y3: O, S, vorzugsweise O,
bedeutet, deren Stereoisomere, Tautomere und deren physiologisch verträglichen Salze oder Einschlussverbindungen.

Ganz besonders bevorzugt sind die Verbindungen, deren Stereoisomere, Tautomere und deren physiologisch verträglichen Salze oder Einschlussverbindungen, ausgewählt aus der Gruppe bestehend aus den Verbindungen der Beispiele und den Verbindungen, die Kombinationen der verschiedenen Substituenten der Verbindungen der Beispiele aufweisen.

Bevorzugt sind außerdem Arzneimittel enthaltend obige Verbindungen der Formel I oder II neben den üblichen Träger und Hilfsstoffen.

Bevorzugt sind auch die oben genannten Arzneimittel in Kombination mit weitere Wirkstoffen zur Tumorbehandlung.

Diese erfindungsgemäßen Verbindungen werden zur Herstellung von Arzneimitteln zur Behandlung von Tumoren, insbesondere von solchen, die durch die Inhibierung der Topoisomerasen I und/oder II behandelt werden können, verwendet. Tumoren, die mit den erfindungsgemäßen Substanzen behandelt werden können sind z.B. Leukemie, Lungenkrebs, Melanome, Prostatatumore und Colontumore.

Des weiteren werden die erfindungsgemäßen Verbindungen zur Herstellung von Arzneimitteln zur Behandlung von Neurodermitis, Parasiten und zur Immunsuppression verwendet.

In der Beschreibung und den Ansprüchen gelten für die einzelnen Substituenten folgende Definitionen:
Der Term "Alkyl" für sich oder als Teil eines anderen Substituenten bedeutet ein lineares oder verzweigtes Alkylketten-Radikal der jeweils angegebenen Länge und optional eine CH₂-Gruppe durch eine Carbonylfunktion ersetzt sein kann. So bedeutet C₁₋₄-Alkyl z.B. Methyl, Ethyl, 1-propyl, 2-Propyl, 2-Methyl-2-propyl, 2-Methyl-1-propyl, 1-Butyl, 2-Butyl, C₁₋₆-Alkyl z.B. C₁₋₄-Alkyl, Pentyl, 1-Pentyl, 2-Pentyl, 3-Pentyl, 1-Hexyl, 2-Hexyl, 3-Hexyl, 4-Methyl-1-pentyl oder 3,3-Dimethyl-butyl.

Der Term "C₁-C₆-Alkylhydroxy" für sich oder als Teil eines anderen Substituenten bedeutet ein lineares oder verzweigtes Alkylketten-Radikal der jeweils angegebenen Länge, das gesättigt oder ungesättigt sein kann und eine OH Gruppe trägt, z.B. Hydroxymethyl, Hydroxyethyl, 1-Hydroxypropyl, 2-Hydroxypropyl.

Der Term "Alkenyl" für sich oder als Teil eines anderen Substituenten bedeutet ein lineares oder verzweigtes Alkylketten-Radikal mit eine oder mehreren C=C-Doppelbindungen der jeweils angegebenen Länge, wobei mehrere Doppelbindungen bevorzugt konjugiert sind. So bedeutet C₂₋₆-Alkenyl z.B. Ethenyl, 1-Propenyl, 2-Propenyl, 2-Methyl-2-propenyl, 2-Methyl-1-propenyl, 1-Butenyl, 2-Butenyl, 1,3-Butdienyl, 2,4-Butdienyl, 1-Pentenyl, 2-Pentenyl, 3-Pentenyl, 1,3-Pentdienyl, 2,4-Pentdienyl, 1,4-Pentdienyl, 1-Hexenyl, 2-Hexenyl, 1,3-Hediexyl, 4-Methyl-1-pentenyl oder 3,3-Dimethylbutenyl.

Der Term "Halogen" steht für Fluor, Chlor, Brom, Jod, bevorzugt Brom und Chlor.

Der Term "NR21R22" steht für eine Dialkylaminogruppe, wobei die beiden Alkylgruppen zusammen mit dem N auch einen 5- oder 6-gliedrigen Ring bilden können.

Ergeben R5 und R6 zusammen mit X₁-C-C-X₂ einen 5, 6 oder 7 gliedrigen Ring, dann sind R5 und R6 zusammen bevorzugt CH₂, CH₂-CH₂, CH=CH, CH₂-CH₂-CH₂, CH=CH-CH₂ oder CH₂-CH=CH

Der Term "Cycloalkyl" für sich oder als Teil eines anderen Substituenten beinhaltet gesättigte, cyclische Kohlenwasserstoffgruppen, mit 3 bis 8 C-Atomen wie z.B. Cyclopropyl, Cyclobutyl, Cyclopentyl, Cyclohexyl, 4-Methyl-cyclohexyl, Cyclohexylmethylen, Cycloheptyl oder Cyclooctyl.

Der Term "Heterocycloalkyl" für sich oder als Teil eines anderen Substituenten beinhaltet Cycloalkylgruppen worin bis zu zwei CH₂-Gruppen durch Sauerstoff-, Schwefel- oder Stickstoffatome ersetzt sein können und eine weitere CH₂-gruppe durch eine Carbonylfunktion ersetzt sein kann, z.B. Pyrrolidin, Piperidin, Morpholin oder

| | | |
|---|---|---|
| 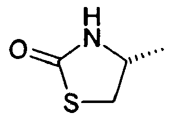 | 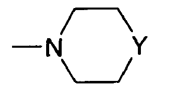 Y=CH2, S, O, NH, NC1-C6-Alkyl | 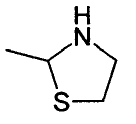 |

Der Term "Aryl" für sich oder als Teil eines anderen Substituenten beinhaltet aromatische Ringsysteme mit bis zu 3 Ringen, bei denen mindestens 1 Ringsystem aromatisch ist und die mit bis zu 3 Substituenten, bevorzugt bis zu 1 Substituenten, wobei die Substituenten unabhängig voneinander die Bedeutung C₁-C₆-Alkyl, OH, NO₂, CN, CF₃, OR11, SH, SR11, C₁-C₆-Alkylhydroxy, C₁-C₆-Alkyl-OR11, COOH, COOR11, NH₂, NHR11, NR11R12, Halogen haben können, wobei die Reste R11, R12 unabhängig von einander C₁-C₁₀-Alkyl, Cycloalkyl, C₁-C₄-Alkyl-Cycloalkyl bedeuten können.

Bevorzugte Aryle sind neben Phenyl und 1-Naphtyl und 2-Naphtyl:

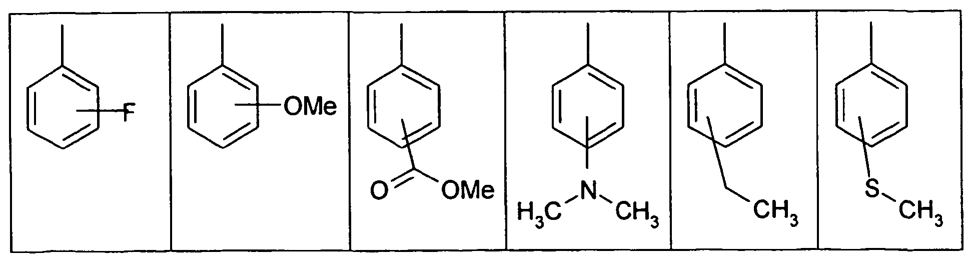

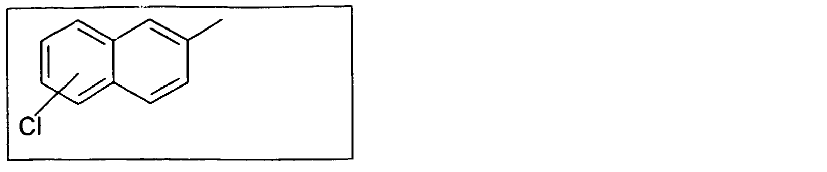

Der Term "Heteroaryl" für sich oder als Teil eines anderen Substituenten beinhaltet aromatische Ringsysteme mit bis zu 3 Ringen, und bis zu 3 gleichen oder verschiedenen Heteroatomen N, S, O bei denen mindestens 1 Ringsystem aromatisch ist und die mit bis zu 3 Substituenten, bevorzugt bis zu 1 Substituenten, wobei die Substituenten unabhängig voneinander die Bedeutung C₁-C₆-Alkyl, OH, NO₂, CN, CF₃, OR11, SH, SR11, C₁-C₆-Alkylhydroxy, C₁-C₆-Alkyl-OR11, COOH, COOR11, NH₂, NHR11, NR11R12, Halogen haben können, wobei die Reste R11 unabhängig von einander die oben angegebenen Bedeutungen haben können.

Bevorzugte Heteroaryle sind:

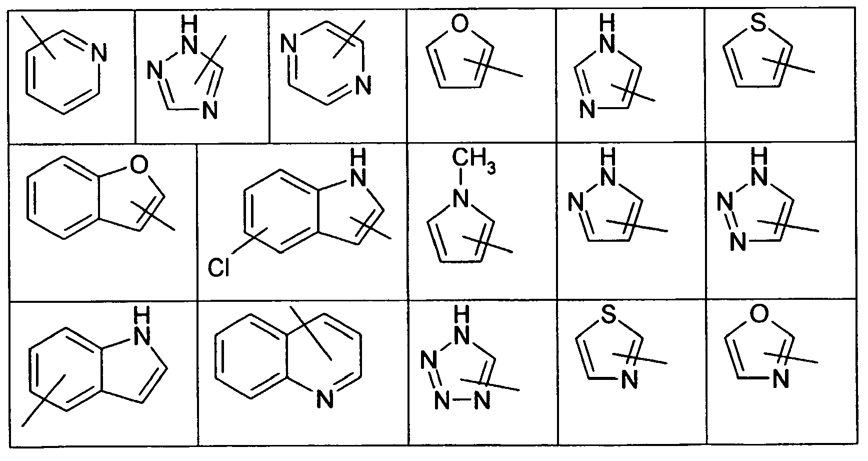

Der Term "Ringsystem" bezieht sich im Allgemeinen auf 3, 4, 5, 6, 7, 8, 9 oder 10 gliedrige Ringe. Bevorzugt sind 5 und 6 gliedrige Ringe. Des weiteren sind Ringsysteme mit einem oder 2 anellierten Ringen bevorzugt.

Die Verbindungen der Formel I können als solche oder falls sie acidische oder basische Gruppen aufweisen in Form ihrer Salze mit physiologisch verträglichen Basen oder Säuren vorliegen. Beispiele für solche Säuren sind: Salzsäure, Zitronensäure, Trifluoressigsäure, Weinsäure, Milchsäure, Phosphorsäure, Methansulfonsäure, Essigsäure, Ameisensäure, Maleinsäure, Fumarsäure, Bernsteinsäure, Hydroxybernsteinsäure, Schwefelsäure, Glutarsäure, Asparaginsäure, Brenztraubensäure, Benzoesäure, Glucuronsäure, Oxalsäure, Ascorbinsäure und Acetylglycin. Beispiele für Basen sind Alkaliionen, bevorzugt Na, K, Erdalkaliionen, bevorzugt Ca, Mg, Ammoniumionen.

Die erfindungsgemäßen Verbindungen können in üblicher Weise oral verabfolgt werden. Die Applikation kann auch i.v., i.m., mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis pro Person zwischen etwa 0.1 µg/kg und 1 g/Kg bei oraler Gabe. Diese Dosis kann in 2 bis 4 Einzeldosen oder einmalig am Tag als Slow-release-Form gegeben werden.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Lösungen, oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1978). Die so erhaltenen Applikationsformen enthalten den Wirkstoff normalerweise in einer Menge von 0,1 bis 99 Gew.-%.

### Experimenteller Teil

Fredericamycin A ist fermentativ oder totalsynthetisch nach den bekannten Methoden zugänglich. Die reduzierten Formen der Formel I b und II b lassen sich durch milde Reduktionsmittel aus den entsprechenden Verbindungen der Formel I a und II a herstellen.

### Herstellung der Substanzen

Für die Synthese von wasserlöslichen Fredericamycin Derivaten wurde Fredericamycin (1) zunächst mit Osmium(IV)oxid an der Dienseitenkette hydroxyliert. (s. Schema 1 ). 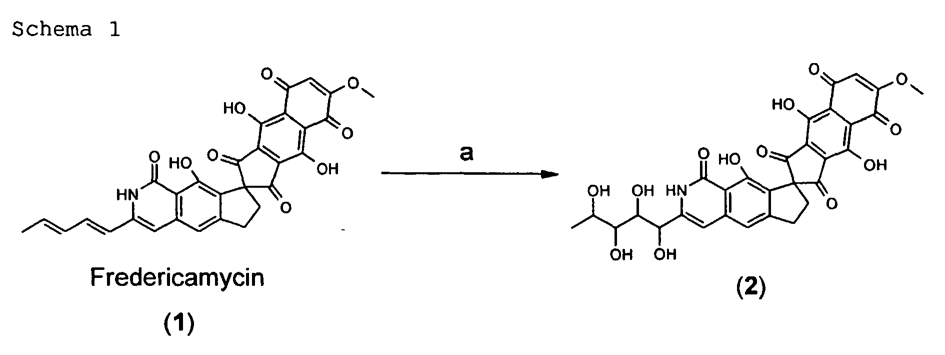
a) OsO₄, N-Methylmorpholin-N-oxid, CH₂Cl₂,CH₃OH,H₂O

Das Fredericamycin-tetrol (2) dient als wichtige Zwischenstufe für die Synthese weiterer Fredericamycin Derivate mit erhöhtem Löslichkeit und/oder Wirkprofil. Durch Jodatspaltung mit Natriummetaperjodat bzw. trägergebundenem Perjodat läßt sich die Tetrolseitenkette in sehr hohen Ausbeuten zum Fredericamycin-aldehyd (4) abbauen (s. Schema 2). 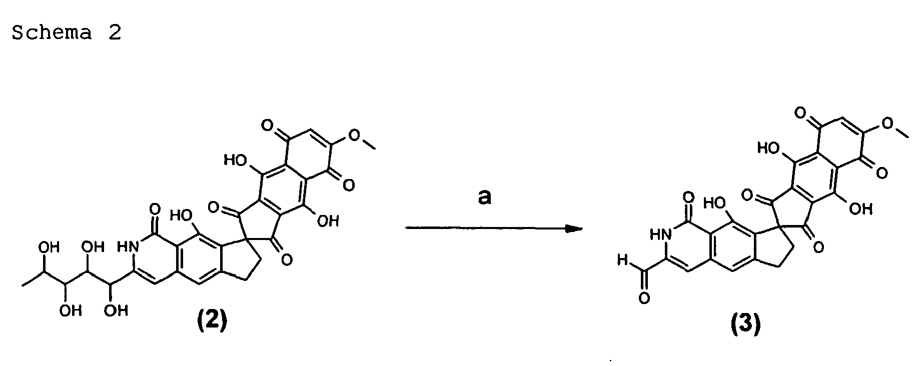
a) NaIO₄-H₂O-DMF oder trägergebundenes-IO₄-H₂O-DMF

Der Fredericamycin-Aldehyd (3) läßt sich mit Acylhydrazonen, Hydroxylamin und O-Alkylhydroxylamine in die entsprechenden Hydrazone ( s. Schema 3 ), bzw. Oxim und Oximether ( s. Schema 4 ) umsetzen. Die Reaktion kann bei Raumtemperatur in Lösungsmittel wie DMF oder Pyridin durchgeführt werden und ist nach wenigen Minuten bis Stunden beendet.

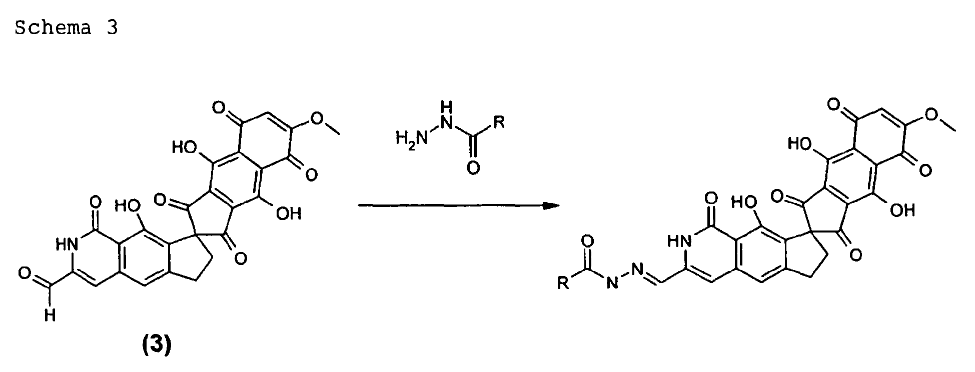

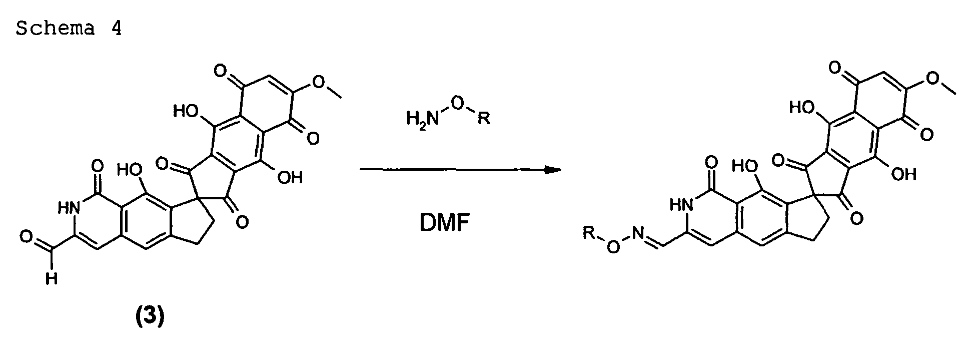

### Halogensubstituierte Fredericamycinderivate R=I,Br,Cl,F

Fredericamycin (1) lässt sich mit Halogenierungsmitteln wie N-Bromsuccinimid (NBS) und N-Jodsuccinimid (NIS) in guten Ausbeuten zu den substituierten 5-Brom- bzw. 5-Jod Fredericamycin Derivaten (4) und (5) umsetzen ( Schema 5).

Die ensprechende Fluorverbindung ist ebenfalls zugänglich. 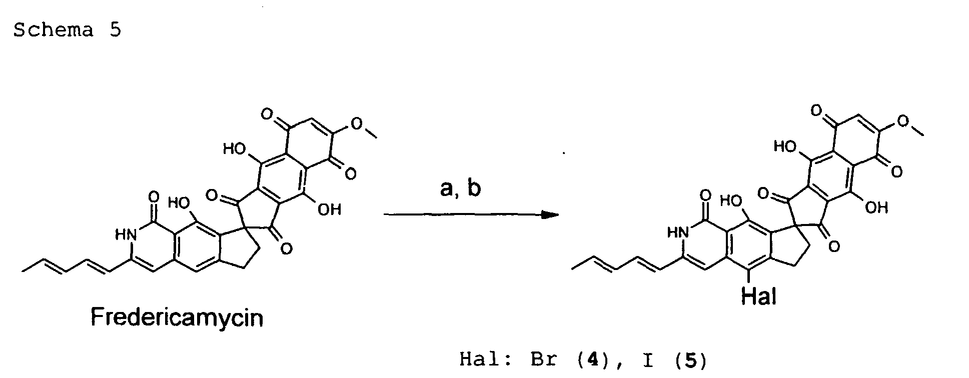
a) N-Bromsuccinimid, DMF, 0°C;
b) N-Jodsuccinimid, DMF, 0°C

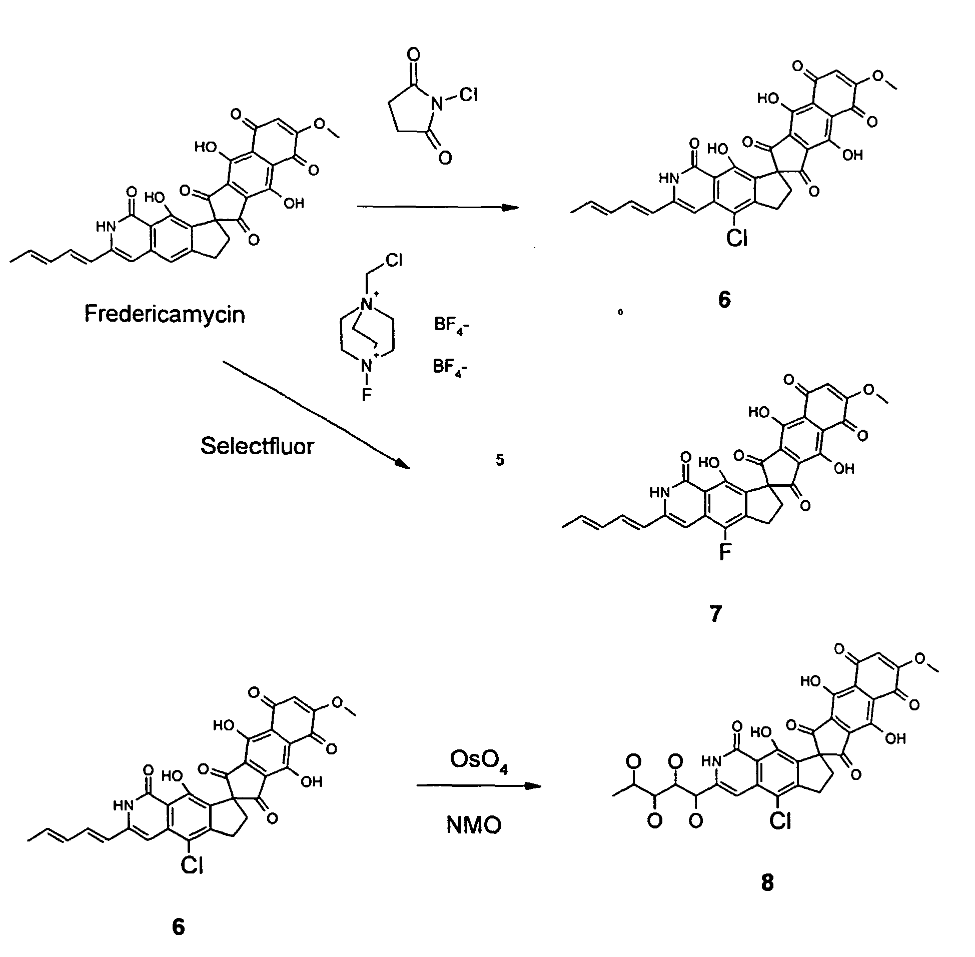

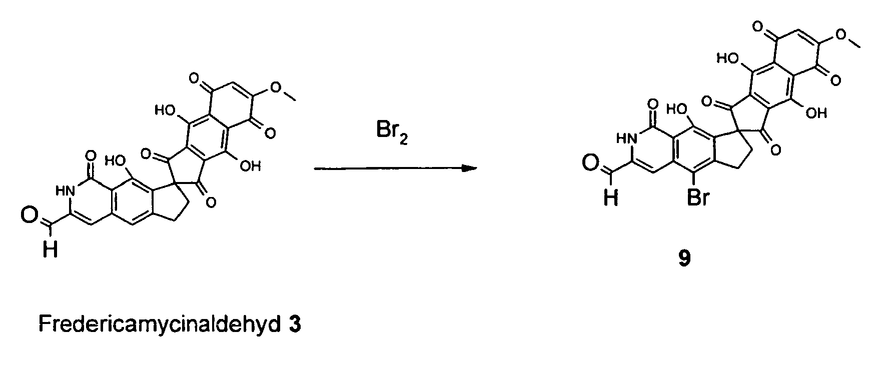

Die hier genannten Fredericamycin Derivate können dann durch Umsetzungen mit den entsprechenden S oder N Nukleophilen zu den beanspruchten Verbindungen umgesetzt werden s. Schema 6.

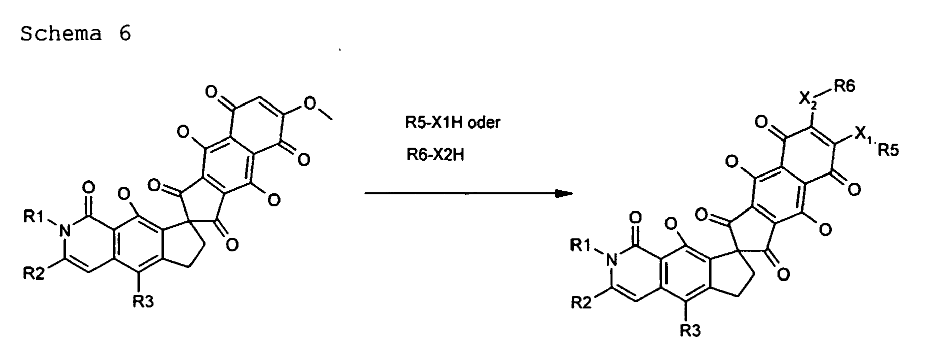

Die Substitutionen von Y1 und oder Y2 gleich N-R5 sind über entsprechende primäre Amine HN-R5 zugänglich.

### Synthesebeispiele:

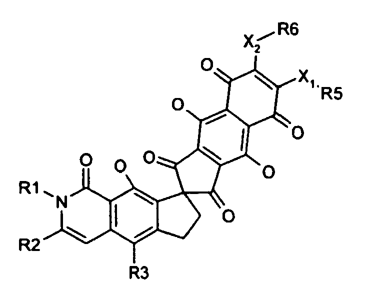

**Tabelle 1**

| **R3** | **X1-R5** | **X2-R6** | **Beispiel** |
|---|---|---|---|
| H | OMe | SCH2COOEt | 1 |
| H | OH | SCH2CH2NEt2 | 2 |
| H | OMe | SCH2CH2OH | 3 |
| H | OMe | SCH2CH2NEt2 | 4 |
| Cl | OMe | SCH2Ph | 5 |
| H | OMe | OH | 6 |

### Herstellung thioanaloger Fredericamycin Derivate

Durch Beschwefelung von Fredericamycin oder seine Derivate mit Lawesson Reagenz oder P₄S₁₀ in Pyridin sind die Thiopyridon analogen Derivate zugänglich (s. Schema 7, dort gezeigt am Fredericamycin A)

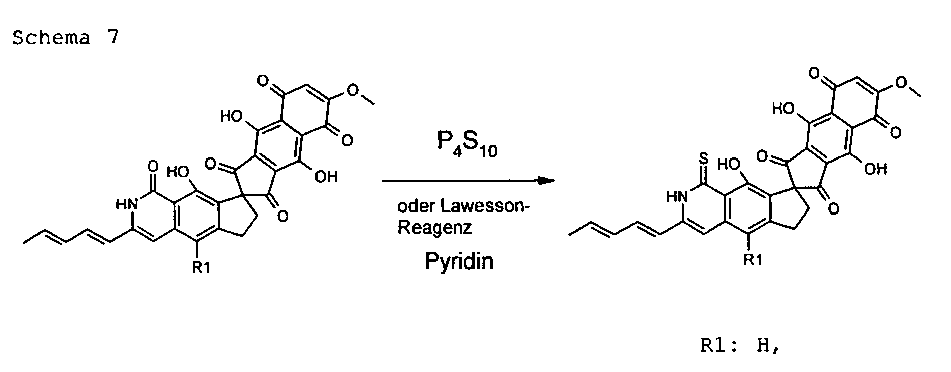

### Biologische Aktivität gegen 12 Krebs Zell-Linien:

LCL(H460/Lunge), MACL(MCF7,Brust), LXFL(529L,Lunge), LXFA(629L,Lunge), MEXF(462NL,Melanom), MEXF(514L,Melanom), MAXF(401NL,Brust), RXF(944L,Renal), RXF(486L,Renal), UXF(1138L,Uterus), PRXF(PC3M,Prostata), PRXF(22RV1).

Wirksamkeit (IC70) gemittelt über alle Zell-Linien in µg/ml bei 5 Testkonzentrationen

**Tabelle 7**

| Beispiel/Referenz | IC70 µg/ml |
|---|---|
| Adriamycin | 0.0210 |
| Cis-Platin | 37.1020 |
| Fredericamycin | 0.2790 |
| 3 | 0.1340 |

### Beispiele gemäß Tabelle 1

### Beispiel 1

### (8S) -4',9.9'-trihydroxy-6-methoxy-7-ethylthioaceto-3-[(1E,3E)-penta-1,3-dienyl]-6,7-dihydrospiro[cyclopenta[g]isoquinoline-8,2'-cyclopenta[b]naphtalene]-1,1'-3',5',8' (2H)-pentone

10mg (18.6µmol) Fredericamycin wird unter Argon in 1ml DMF gelöst und anschließend bei Raumtemperatur mit 2.5µl (22.3µmol) Mercaptoessigsäureethylester versetzt. Nach 24h hat sich laut HPLC (RP18, Acetonitril/Wasser) ein einheitliches neues Produkt gebildet. Man engt die Reaktionsmischung im Hochvakuum zur Trockene ein.

Rote Kristallmasse Ausbeute: 12mg (98%). M/e=558.9 (M+H), λmax=510nm

### Beispiel 2

### (8S)-4',9.9'-trihydroxy-6-hydroxy-7 (2-diethylaminoethylmercapto)-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone

10mg (18.6µmol) Fredericamycin wird unter Argon in 1ml DMF gelöst und anschließend bei Raumtemperatur mit 3.8mg (22.3µmol) 2-Diethylaminoethanthiol.HCl verzetzt. Nach 23h wurden weitere 3.17mg 2-Diethylaminoethanthiol.HCl zugegeben. Man engt nach 45h gesamter Reaktionszeit die Reaktionsmischung im Hochvakuum zur Trockene ein und chromatographiert den Rückstand mittels präparativer HPLC ( RP18, Acetonitril-Wasser ).

Rote Kristallmasse Ausbeute: 4mg ( 33% ). M/e=657.5 (M+H), λmax=486nm

### Beispiel 3

### (8S)-4',9.9'-trihydroxy-6-methoxy-7(2-hydroxyethylmercapto)-3 [(1E,3E)-penta-1,3-dienyl]-6,7-dihydrospiro[cyclopenta[g] isoquinoline-8,2'-cyclopenta[b]naphtalene]-1,1'-3',5',8'(2H)-pentone

10mg ( 18.6µmol ) Fredericamycin wird unter Argon in 1ml DMF gelöst und anschließend bei Raumtemperatur mit 1.6µl (22.3µmol) Mercaptoethanol versetzt. Nach 20h hat sich laut HPLC ( RP18, Acetonitril/Wasser ) ein einheitliches neues Produkt gebildet. Man engt die Reaktionsmischung im Hochvakuum zur Trockene ein.

Rote Kristallmasse Ausbeute: 11mg ( 99% ). M/e=617.4 (M+H), λmax=486nm

### Beispiel 4

### (8S)-4',9.9'-trihydroxy-6-methoxy-7-(2-diethylaminoethylmercapto)-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone

10mg ( 18.6µmol ) Fredericamycin wird unter Argon in 1ml DMF gelöst und anschließend bei Raumtemperatur mit 3.8mg (22.3µmol) 2-Diethylaminoethanthiol.HCl versetzt. Nach 6h wurden weitere 1.9mg 2-Diethylaminoethanthiol.HCl zugegeben. Nach 23h weitere 1.9mg Diethylaminoethanthiol.HCl. Man engt nach insgesamt 30h die Reaktionsmischung im Hochvakuum zur Trockene ein und chromatographiert den Rückstand mittels präparativer HPLC ( RP18, Acetonitril-Wasser ).

Rote Kristallmasse Ausbeute: 10mg ( 80% ). M/e=671.4 (M+H), λmax=486nm

### Beispiel 5

### (8S)-4',9.9'-trihydroxy-6-methoxy-7-(benzylmercapto)-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone

5.0mg ( 8.71µmol ) 5-Chlor Fredericamycin wird unter Argon in 1ml DMF gelöst und anschließend bei Raumtemperatur mit 1.23µl (10.45µmol) Benzylmercaptan versetzt. Nach 4h engt man die Reaktionsmischung im Hochvakuum zur Trockene ein.

Rote Kristallmasse Ausbeute: 6mg ( 99% ). M/e=695.9 (M+H), λmax=504nm

### Beispiel 6

### (8S)-4',9.9'-trihydroxy-6-methoxy-7-hydroxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone

10mg ( 18.6pmol ) Fredericamycin wird unter Argon in lml DMF gelöst und anschließend bei Raumtemperatur mit 2.5mg (22.3µmol) 2-Aminoethanthiol.HCl versetzt. Nach 26h wurden weitere 2.1mg 2-Aminoethanthiol.HCl und etwas Trifluoressigsäure zugegeben. Nach insgesamt 72h engt man im Hochvakuum zur Trockene ein und chromatographiert den Rückstand mittels präparativer HPLC ( RP18, Acetonitril-Wasser ).

Rote Kristallmasse Ausbeute: 9mg ( 87% ). M/e=554.5 (M-H), λmax=372nm

## Patentansprüche

1. Verbindungen gemäß allgemeiner Formel Ia oder Ib : 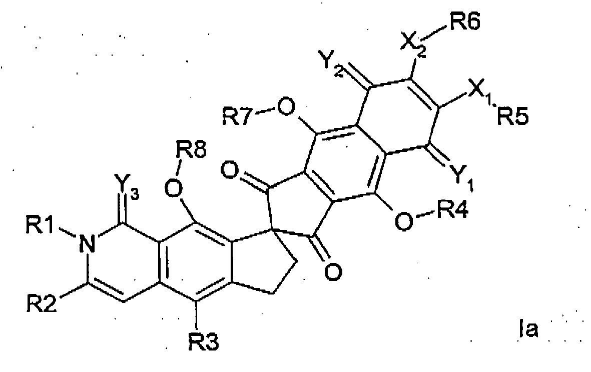 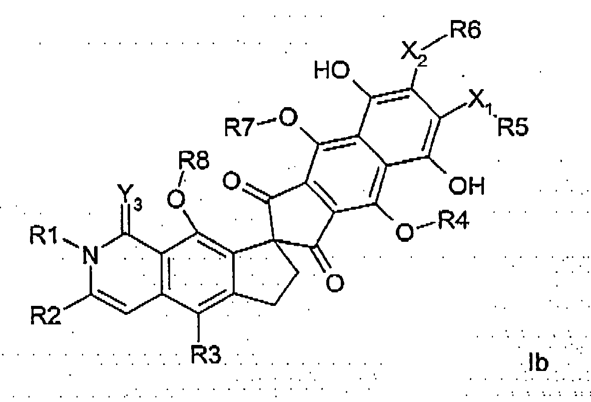 wobei jeweils,
R1 H, C₁-C₆Alkyl, C₃-C₈Cycloalkyl,C₁-C₄Alkylcycloalkyl,
R2 C₁-C₁₄ Alkyl, C₁-C₁₄ Alkenyl, C₁-C₄ Alkylaryl, Heteroaryl und zwar aromatische Ringsysteme mit bis zu 3 Ringen, und bis zu 3 gleichen oder verschiedenen Heteroatomen N, S, O bei denen mindestens 1 Ringsystem aromatisch ist und die mit bis zu 3 Substituenten, wobei die Substituenten unabhängig voneinander die Bedeutung C1-C6-Alkyl, OH, NO₂, CN, CF₃, OR11, SH, SR11, C₁-C₆-Alkylhydroxy,C₁-C₆-Alkyl-OR11, COOH,COOR11, NH₂, NHR11, NR11R12, Halogen haben können, wobei die Reste R11, R12 unabhängig voneinander C₁-C₁₀-Alkyl, C₃-C₈ Cycloalkyl, C₁-C₄-Alkyl-Cycloalkyl bedeuten können, C₁-C₄ Alkylheteroaryl, C₃-C₈ Cycloalkyl, C₁-C₄ Alkylcycloalkyl, Heterocycloalkyl und zwar Cycloalkylgruppen wobei bis zu zwei CH₂- Gruppen durch Sauerstoff-, Schwefel- oder Stickstoffatome ersetzt sein können und eine weitere CH₂- Gruppe durch eine Carbonylfunktion ersetzt sein kann, C₁-C₄ Alkylheterocycloalkyl, CₘH₂ₘ₊ₒ₋ₚYₚ (mit m = 1 to 6, für o = 1, p =1 to 2m+o; für m = 2 to 6, o = -1, p = 1 to 2m+o; für m = 4 to 6; o = -2, p = 1 to 2m+o; Y = unabhängig voneinander ausgewählt aus der Gruppe Halogen, OH, OR21, NH₂, NHR21, NR21R22, SH, SR21), CH₂NHCOR21, CH₂NHCSR21, CH₂S(O)nR21, mit n = 0, 1, 2, CH₂SCOR21, CH₂OSO₂-R21, CHO, CH=NOH, CH(OH)R21, -CH=NOR21,-CH=NOCOR21, -CH=NOCH₂CONR21R22, -CH=NOCH(CH₃)CONR21R22,-CH=NOC(CH₃)₂CONR21R22, -CH=N-NHCO-R23, -CH=N-NHCO-CH₂NHCOR21,-CH=N-O-CH₂NHCOR21, -CH=N-NHCS-R23, -CH=CR24R25 (trans oder cis), COOH, COOR21, CONR21R22, -CH=NR21, -CH=N-NR21R22, 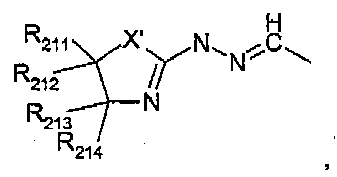 mit X'=NR215, O, S, and R211, R212, R213, R214, R215 unabhängig voneinander H oder C₁-C₆ Alkyl), -CH=N-NHSO₂ Aryl, -CH=N-NHSO₂ Heteroaryl,
R21, R22 unabhängig voneinander C₁-C₁₄ Alkyl, C₁-C₁₄ Alkanoyl, C₁-C₆ Alkylhydroxy, C₁-C₆; Alkylamino, C₁-C₆Alkylamino-C₁-C₆ Alkyl, C₁-C₆ Alkylamino-di-C₁-C₆ alkyl, C₃-C₈ Cycloalkyl, C₁-C₄ Alkylcycloalkyl, Heterocycloalkyl, C₁-C₄ Alkylheterocycloalkyl, Aryl und zwar aromatische Ringsysteme mit bis zu 3 Ringen, bei denen mindestens 1 Ringsystem aromatisch ist und die mit bis zu 3 Substituenten, bevorzugt bis zu 1 Substituenten, wobei die Substituenten unabhängig voneinander die Bedeutung C₁-C₆-Alkyl, OH, NO₂, CN, CF₃, OR11, SH, SR11, C₁-C₆-Alkylhydroxy, C₁-C₆-Alkyl-OR11, COOH, COOR11, NH2, NHR11, NR11R12, Halogen haben können, wobei die Reste R11, R12 unabhängig von einander C₁-C₁₀-Alkyl, C₃-C₈ Cycloalkyl, C₁-C₄-Alkyl-Cycloalkyl bedeuten können, Aryloyl, C₁-C₄ Alkylaryl, Heteroaryl wie in R2 definiert, Heteroaryloyl, C₁-C₄ Alkylheteroaryl, Cycloalkanol, C₁-C₄ Alkanoylcycloalkyl, Heterocycloalkanoyl, C₁-C₄ Alkanoylheterocycloalkyl, C₁-C₄ Alkanoylaryl, C₁-C₄ Alkanoylheteroaryl, Mono-und Di-Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschliesslich ihren Desoxyverbindungen,
R23 unabhängig von R21, dieselben Bedeutungen wie R21, oder CH₂-pyridiniumsalze, CH₂-tri-C₁-C₆ Alkylammoniumsalze,
R24 unabhängig von R21, dieselben Bedeutungen wie R21, oder H, CN, NOCH₃, COOH, COOR21, CONR21R22, NH₂, NHCOR21,
R25 unabhängig von R21, dieselben Bedeutungen wie R21, oder H, CN, COCH₃, COOH, COOR21, CONR21R22, NH₂, NHCOR21,
R24, R25 zusammen C₄-C₈ Cycloalkyl,
R3 H, F, Cl, Br, I, OH, OR31, NO₂, NH₂, NHR31, NR31R32, NHCHO, NHCOR31, NHCOCF3, CH₃₋ₘHalₘ (mit Hal = Cl, F, insbesondere F, and m = 1, 2, 3), OCOR31,
R31, 32 unabhängig voneinander C₁-C₆ Alkyl,
R5, R6 unabhängig voneinander H, C₁-C₁₄ Alkyl, C₂-C₁₄ Alkenyl, Aryl wie in R21 definiert, C₁-C₄ Alkylaryl, Heteroaryl wie in R2 definiert, C₁-C₄ Alkylheteroaryl, C₃-C₈ Cycloalkyl, C₁-C₄ Alkylcycloalkyl, Heterocycloalkyl wie in R2 definiert, C₁-C₄ Alkylheterocycloalkyl, CₘH₂ₘ₊ₒ₋ₚYₚ (mit m=1 to 6, für o=1, p = 1 to 2m+o; für m = 2 to 6, o = -1, p = 1 to 2m+o; für m = 4 to 6, o = -2, p = 1 to 2m+o; Y = unabhängig voneinander ausgewählt aus der Gruppe Halogen, OH, OR21, NH₂, NHR21, NR21R22, SH, SR21), oder R5 and R6 ergeben zusammen mit X₁-C-C-X₂ einen 5, 6, oder 7 gliedrigen Ring,
R4, R7, R8 unabhängig voneinander H, C₁-C₆ Alkyl, CO-R41,
R41 unabhängig von R21, dieselben Bedeutungen wie R21,
X1 O, S, NH, N-C₁-C₈ Alkyl, N-Cycloalkyl,
X2 O, S, NH, Alkyl, N-Cycloalkyl,
Y1 O, N-R9, wobei R9 unabhängig von R5, dieselben Bedeutungen wie R5 annehmen kann,
Y2 O, N-R10, wobei R10 unabhängig von R5, dieselben Bedeutungen wie R5 annehmen kann,
und, falls Y1 oder Y2 gleich N-R9 oder N-R10, kann X2-R6 gleich H,
Y3 O, S, NH,
bedeutet, deren Stereoisomere, Tautomere und deren physiologisch verträglichen Salze oder Einschlussverbindungm.

2. Verbindungen gemäß Anspruch1, wobei Formel I a oder I b, die Stereochemie von Formel II a oder II b 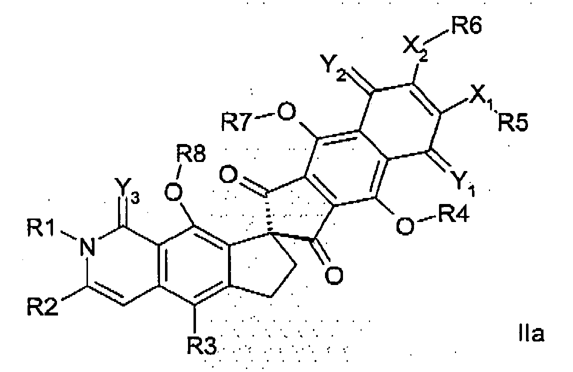 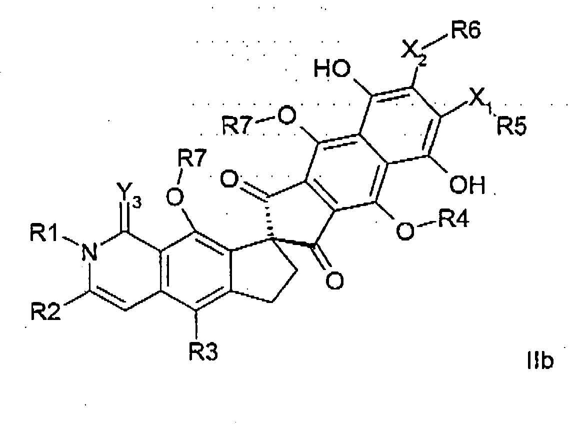 annimmt.

3. Verbindungen gemäß einem der Ansprüche 1 bis 3, wobei
R1 H, C₁-C₅Alkyl, C₃-C₈ Cycloalkyl,
R2 C₁-C₅ Alkyl, C₁-C₄ Alkylaryl, C₂-C₅ Alkenyl, Heteroaryl und zwar aromatische Ringsysteme mit bis zu 3 Ringen, und bis zu 3 gleichen oder verschiedenen Heteroatomen N, S, O bei denen mindestens 1 Ringsystem aromatisch ist und die mit bis zu 3 Substituenten, wobei die Substituenten unabhängig voneinander die Bedeutung C1-C6-Alkyl, OH, NO₂, CN, CF₃, OR11, SH, SR11,C₁-C₆-Alkylhydroxy,C₁-C₆-Alkyl-OR11, COOH,COOR11, NH₂, NHR11, NR11R12, Halogen haben können, wobei die Reste R11, R12 unabhängig voneinander C₁-C₁₀-Alkyl, C₃-C₈ Cycloalkyl, C₁-C₄-Akyl-Cycloakyl bedeuten können, C₁-C₄ Alkylheteroaryl, CHF₂, CF₃, Polyolseitenkette, CHOH-CHOH-CHOH-CHOH-CH₃, CHOH-CHOH-CH=CH-CH₃, CH=CH-CHOH-CHOH-CH₃, CH₂Y (Y = F, Cl, Br, I), CH₂NH₂, CH₂NR21R22, CH₂NHCOR23, CH₂NHCSR23, CH₂SH, CH₂S(O)nR21, mit n = 0, 1, 2, CH₂SCOR21, CH₂OH, CH₂OR21, CH₂OSO₂-R21, CHO, CH(OR21)₂, CH(SR21)₂, CN, CH=NOH, CH=NOR21, CH=NOCOR21, CH=N-NHCO-R23, CH=CR24, R25 (trans oder cis), COOH (insbesondere deren physiologisch verträglichen Salze), COOR21, CONR21R22, -CH=NR21, -CH=N-NR21R22, 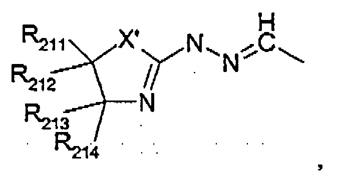 (mit X'= NR215, O, S, and R211, R212, R213, R214, R215 unabhängig voneinander H oder C₁-C₆ Alkyl), -CH=N-NHSO₂ Aryl, -CH=N-NHSO₂ - Heteroaryl, CH=N-NHCO-R23,
R21, R22 unabhängig voneinander C₁-C₆ Alkyl, C₃-C₈ Cycloalkyl, Aryl und zwar aromatische Ringsysteme mit bis zu 3 Ringen, bei denen mindestens 1 Ringsystem aromatisch ist und die mit bis zu 3 Substituenten, bevorzugt bis zu 1 Substituenten, wobei die Substituenten unabhängig voneinander die Bedeutung C₁-C₆-Alkyl, OH, NO₂, CN, CF₃, OR11, SH, SR11, C₁-C₆-Alkylhydroxy, C₁-C₆-Alkyl-OR11, COOH, COOR11, NH2, NHR11, NR11R12, Halogen haben können, wobei die Reste R11, R12 unabhängig von einander C₁-C₁₀-Alkyl, C₃-C₈ Cycloalkyl, C₁-C₄-Alkyl-Cycloalkyl bedeuten können, C₁-C₄ Alkylaryl, Heteroaryl wie in R2 definiert, C₁-C₄ Alkylheteroaryl,
R23 unabhängig von R21, dieselben Bedeutungen wie R21, oder CH₂-pyridiniumsalze, CH₂-tri-C₁-C₆, Alkylammoniumsalze,
R24 unabhängig von R21, dieselben Bedeutungen wie R21, oder H, CN, COCH₃, COOH, COOR21, CONR21R22, NH₂, NHCOR21,
R25 unabhängig von R21, dieselben Bedeutungen wie R21, oder H, CN, COCH₃, COOH, COOR21, CONR21R22, NH₂, NHCOR21,
R24, R25 zusammen C₄-C₈ Cycloalkyl,
R3 F, Cl, Br, I, NO₂, NH₂, NHCOR31,
R31 unabhängig voneinander C₁-C₆ Alkyl.
R5, R6 H, unabhängig voneinander C₁-C₁₄ Alkyl, C₂-C₁₄ Alkenyl, Aryl wie in R21 definiert, C₁-C₄ Alkylaryl, Heteroaryl wie in R2 definiert, C₁-C₄ Alkylheteroaryl, C₃-C₈ Cycloalkyl, C₁-C₄ Alkylcycloalkyl, Heterocycloalkyl und zwar Cycloalkylgruppen wobei bis zu zwei CH₂- Gruppen durch Sauerstoff-, Schwefel- oder Stickstoffatome ersetzt sein können und eine weitere CH₂- Gruppe durch eine Carbonylfunktion ersetzt sein kann, C₁-C₄ Alkylheterocycloalkyl, CₘH₂ₘ₊ₒ₋ₚYₚ (mit m = 1 to 6, für o =1, p = to 2m+o; für m = 2 to 6, o = -1, p = 1 to 2m+o; für m = 4 to 6, o = -2, p = 1 to 2m+o; Y = unabhängig voneinander ausgewählt aus der Gruppe Halogen, OH, OR21, NH₂, NHR21, NR21R22, SH, SR21), oder R5 and R6 ergeben zusammen mit X₁-C-C-X₂ einen 5, 6, oder 7 gliedrigen Ring,
R4, R7, R8 unabhängig voneinander H, C₁-C₆ Alkyl, CO-R41,
R41 unabhängig von R21, dieselben Bedeutungen wie R21,
Y3 O, S, vorzugsweise O,
bedeutet, deren Stereoisomere, Tautomere und deren physiologisch verträglichen Salze oder Einschlussverbindungen.

4. Verbindungen gemäss einem der Ansprüche 1 bis 3 in der Form von Einschlussverbindungen mit Cyclodextrin, insbesondere alpha- Cyclodextrin.

5. Arzneimittel enthaltend Verbindungen nach einem der Ansprüche 1 bis 4 neben den üblichen Träger und Hilfsstoffe.

6. Arzneimittel nach Anspruch 5 in Kombination mit weitere Wirkstoffen zur Tumorbehandlung.

7. Verwendung von Verbindungen gemäss einem der Ansprüche 1 bis 4 zur Herstellung von Arzneimitteln zur Behandlung von Tumoren.

8. Verwendung von Verbindungen gemäss einem der Ansprüche 1 bis 4 zur Herstellung von Arzneimitteln zur Behandlung von Parasiten.

9. Verwendung von Verbindungen gemäß einem der Anspruche 1 bis 4 zur Herstellung von Arzneimitteln zur Immunsupression.

10. Verwendung von Verbindungen gemäß einem der Ansprüche 1 bis 4 zur Herstellung von Arzneimitteln zur Behandlung von Neurodermitis.

## Claims

1. The compounds according to the general formula Ia or Ib: 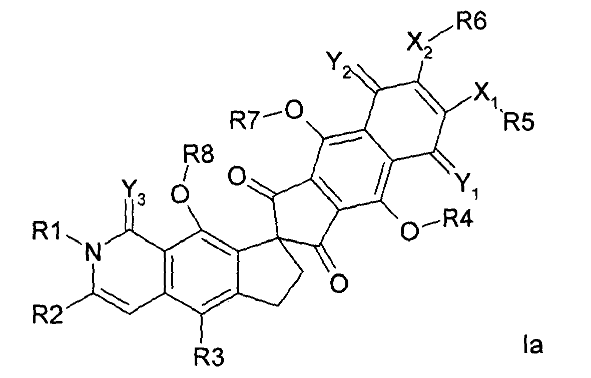 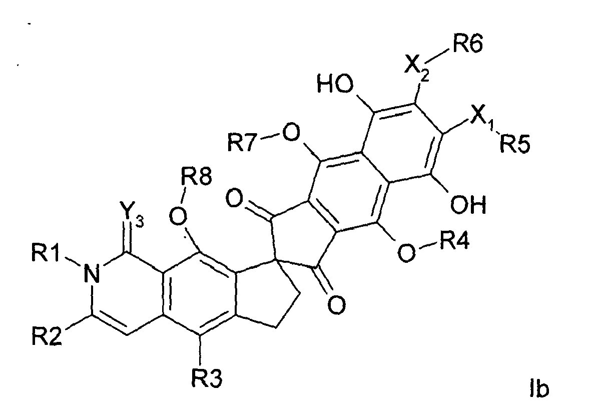 wherein in each,
R1 means H, C1-C₆ alkyl, C₃-C₈ cycloalkyl, C₁-C₄ alkylcycloalkyl,
R2 means C₁-C₁₄ alkyl, C₂-C₁₄ alkenyl, C₁-C₄ alkylaryl, heteroaryl namely aromatic ring systems with up to 3 rings and with up to 3 identical or different heteroatoms N, S, O, in which at least 1 ring system is aromatic, and those with up to 3 substituents, wherein the substituents independently from each other may have the meaning C₁-C₆ alkyl, OH, NO₂, CN, CF₃, OR11, SH, SR11, C₁-C₆ alkylhydroxy, C₁-C₆ alkyl-OR11, COOH, COOR11, NH₂, NHR11, NR11R12, halogen, wherein the residues R11, R12 independently from each other may mean C₁-C₁₀ alkyl, C₃-C₈ cycloalkyl, C₁-C₄ alkylcycloalkyl;
C₁-C₄ alkyl heteroaryl, C₃-C₈ cycloalkyl, C₁-C₄ alkyl-cycloalkyl, heterocycloalkyl namely cycloalkyl groups, wherein up to two CH₂ groups may be substituted by oxygen, sulfur or nitrogen atoms, and another CH₂ group may be substituted by a carbonyl function;
C₁-C4 alkylheterocycloalkyl, CₘH₂ₘ₊ₒ₋ₚYₚ (with m = 1 to 6, for o = 1, p = 1 to 2m+o; for m= 2 to 6, o=-1,p= 1 to 2m+o; for m = 4 to 6, o=-2,p=1 1 to 2m+o; Y = independently from each other selected from the group consisting of halogen, OH, OR21, NH₂, NHR21, NR21R22, SH, SR21), CH₂NHCOR21, CH₂NHCSR21, CH₂S(O)nR21, with n = 0, 1, 2, CH₂SCOR21, CH₂OSO₂-R21, CHO, CH=NOH, CH(OH)R21, -CH=NOR21, -CH=NOCOR21, -CH=NOCH₂CONR21R22,-CH=NOCH(CH₃)CONR21R22, -CH=NOC(CH₃)₂CONR21R22, -CH=N-NHCO-R23,-CH=N-NHCO-CH₂NHCOR21, -CH=N-O-CH₂NHCOR21, -CH=N-NHCS-R23,-CH=CR24R25 (trans or cis), COOH, COOR21, CONR21R22, -CH=NR21, -CH=N-NR21 R22, 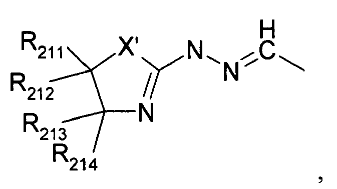 (with X' = NR215, O, S, and R211, R212, R213, R214, R215 being independently from each other H or C₁-C₆ alkyl), -CH=N-NHSO₂ aryl, -CH=N-NHSO₂ heteroaryl,
R21, R22 are independently from each other C₁-C₁₄ alkyl, C₁-C₁₄ alkanoyl, C₁-C₆ alkylhydroxy, C₁-C₆ alkylamino, C₁-C₆ alkylamino-C₁-C₆ alkyl, C₁-C₆ alkylamino-di-C₁-C₆ alkyl, C₃-C₈ cycloalkyl, C₁-C₄ alkylcycloalkyl, heterocycloalkyl, C₁-C₄ alkylheterocycloalkyl, aryl namely aromatic ring systems with up to 3 rings and with up to 3 identical or different heteroatoms N, S, O, in which at least 1 ring system is aromatic, and those with up to 3 substituents, wherein the substituents independently from each other may have the meaning C₁-C₆ alkyl, OH, NO₂, CN, CF₃, OR11, SH, SR11, C₁-C₆ alkylhydroxy, C₁-C₆ alkyl-OR11, COOH, COOR11, NH₂, NHR11, NR11R12, halogen, wherein the residues R11, R12 independently from each other may mean C₁-C₁₀ alkyl, C₃-C₈ cycloalkyl, C₁-C₄ alkylcycloalkyl;
aryloyl, C₁-C₄ alkylaryl, heteroaryl as defined in R2, heteroaryloyl, C₁-C₄ alkylheteroaryl, cycloalkanoyl, C₁-C₄ alkanoylcycloalkyl, heterocycloalkanoyl, C₁-C₄ alkanoylheterocycloalkyl, C₁-C₄ alkanoylaryl, C₁-C₄ alkanoylheteroaryl, mono- and di-sugar residues linked through a C atom which would carry an OH residue in the sugar, wherein the sugars are independently from each other selected from the group consisting of glucuronic acid and its stereo isomers at all optical C-atoms, aldopentoses, aldohexoses, including their desoxy compounds,
R23 independently of R21, has the same meanings as R21, or CH₂-pyridinium salts, CH₂-tri-C₁-C₆ alkylammonium salts,
R24 independently of R21, has the same meanings as R21, or H, CN, COCH₃, COOH, COOR21, CONR21R22, NH₂, NHCOR21,
R25 independently of R21, has the same meanings as R21, or H, CN, COCH₃, COOH, COOR21, CONR21R22, NH₂, NHCOR21,
R24, R25 together mean C₄-C₈ cycloalkyl,
R3 means H, F, Cl, Br, I, OH, OR31, NO₂, NH₂, NHR31, NR31R32, NHCHO, NHCOR31, NHCOCF3, CH₃₋ₘhalₘ (with hal = Cl, F, particularly F, and m = 1,2,3),OCOR31,
R31, 32 independently from each other mean C₁-C₆ alkyl,
R5, R6 independently from each other mean H, C₁-C₁₄ alkyl, C₂-C₁₄ alkenyl, aryl as defined in R21, C₁-C₄ alkylaryl, heteroaryl as defined in R2, C₁-C₄ alkylheteroaryl, C₃-C₈ cycloalkyl, C₁-C₄ alkylcycloalkyl, heterocycloalkyl as defined in R2, C₁-C₄ alkylheterocycloalkyl, CₘH₂ₘ₊ₒ₋ₚYₚ (with m = 1 to 6, for o = 1, p = 1 to 2m+o; for m = 2 to 6, o = -1, p = 1 to 2m+o; for m = 4 to 6, o = -2, p = 1 to 2m+o; Y = independently selected from the group consisting of halogen, OH, OR21, NH₂, NHR21, NR21R22, SH, SR21), or R5 and R6, together with X₁-C-C-X₂, form a ring with 5, 6, or 7 members,
R4, R7, R8 independently from each other mean H, C₁-C₆ alkyl, CO-R41,
R41 independently from R21 has the same meanings as R21,
X1 means O, S, NH, N-C₁-C₈ alkyl, N-cycloalkyl,
X2 means O, S, NH, N-C₁-C₈ alkyl, N-cycloalkyl,
Y1 means O, N-R9, wherein R9 may, independently from R5, adopt the same meanings as R5,
Y2 means O, N-R10, wherein R10 may, independently from R5, adopt the same meanings as R5,
and, if Y1 or Y2 are N-R9 or N-R10, X2-R6 may be H,
Y3 means O, S, NH,
as well their stereoisomers, tautomers, and their physiologically tolerable salts or inclusion compounds.

2. The compounds according to claim 1, wherein Formula Ia or Ib adopt the stereochemistry of Formula IIa or IIb

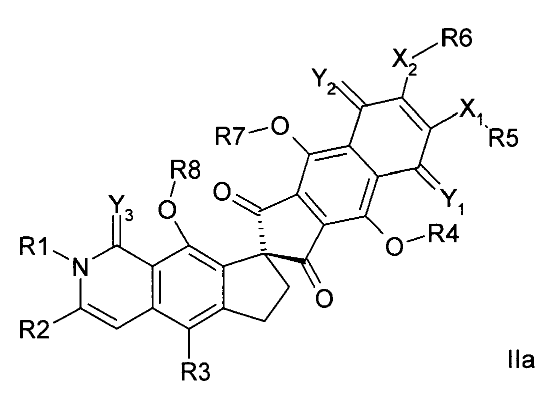

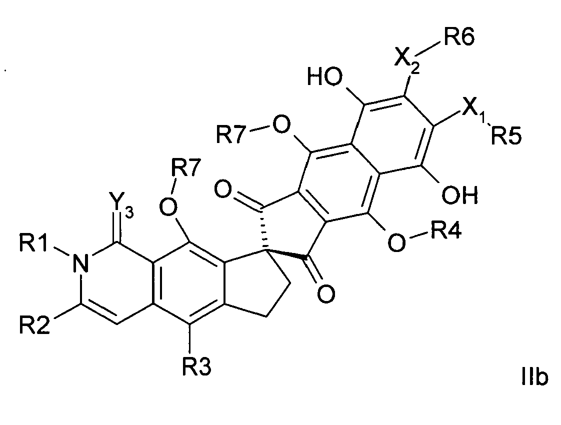

3. The compounds according to one of the claims 1 to 2, wherein
R1 means H, C₁-C₅ alkyl, C₃-C₈ cycloalkyl,
R2 means C₁-C₅ alkyl, C₁-C₄ alkylaryl, C₂-C₅ alkenyl, heteroaryl namely aromatic ring systems with up to 3 rings and with up to 3 identical or different heteroatoms N, S, O, in which at least 1 ring system is aromatic, and those with up to 3 substituents, wherein the substituents independently from each other may have the meaning C₁-C₆ alkyl, OH, NO₂, CN, CF₃, OR11, SH, SR11, C₁-C₆ alkylhydroxy, C₁-C₆ alkyl-OR11, COOH, COOR11, NH₂, NHR11, NR11R12, halogen, wherein the residues R11, R12 independently from each other may mean C₁-C₁₀ alkyl, C₃-C₈ cycloalkyl, C₁-C₄ alkylcycloalkyl;
C₁-C₄ alkylheteroaryl, CHF₂, CF₃, polyol side chain, CHOH-CHOH-CHOH-CHOH-CH₃, CHOH-CHOH-CH=CH-CH₃, CH=CH-CHOH-CHOH-CH₃, CH₂Y (Y = F, Cl, Br, I), CH₂NH₂, CH₂NR21R22, CH₂NHCOR23, CH₂NHCSR23, CH₂SH, CH₂S(O)nR21, with n = 0, 1, 2, CH₂SCOR21, CH₂OH, CH₂OR21, CH₂OSO₂-R21, CHO, CH(OR21)₂, CH(SR21)₂, CN, CH=NOH, CH=NOR21, CH=NOCOR21, CH=N-NHCO-R23, CH=CR24, R25 (trans or cis), COOH (particularly their physiologically tolerable salts), COOR21, CONR21R22, -CH=NR21, -CH=N-NR21R22, 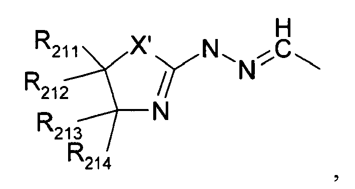 (with X' = NR215, O, S, and R211, R212, R213, R214, R215 being independently from each other H or C₁-C₆ alkyl), -CH=N-NHSO₂ aryl,-CH=N-NHSO₂ heteroaryl, CH=N-NHCO-R23,
R21, R22 independently from each other mean C₁-C₆ alkyl, C₃-C₈ cycloalkyl, aryl namely aromatic ring systems with up to 3 rings and with up to 3 identical or different heteroatoms N, S, O, in which at least 1 ring system is aromatic, and those with up to 3 substituents, wherein the substituents independently from each other may have the meaning C₁-C₆ alkyl, OH, NO₂, CN, CF₃, OR11, SH, SR11, C₁-C₆ alkylhydroxy, C₁-C₆ alkyl-OR11, COOH, COOR11, NH₂, NHR11, NR11R12, halogen, wherein the residues R11, R12 independently from each other may mean C₁-C₁₀ alkyl, C₃-C₈ cycloalkyl, C₁-C₄ alkylcycloalkyl;
C₁-C₄ alkylaryl, heteroaryl as defined in R2, C₁-C₄ alkylheteroaryl,
R23 independently of R21, has the same meanings as R21, or CH₂-pyridinium salts, CH₂-tri-C₁-C₆ alkylammonium salts,
R24 independently of R21, has the same meanings as R21, or H, CN, COCH₃, COOH, COOR21, CONR21R22, NH₂, NHCOR21,
R25 independently of R21, has the same meanings as R21, or H, CN, COCH₃, COOH, COOR21, CONR21R22, NH₂, NHCOR21,
R24, R25 together mean C₄-C₈ cycloalkyl,
R3 means F, Cl, Br, I, NO₂, NH₂, NHCOR31,
R31 independently from each other means C₁-C₆ alkyl,
R5, R6 mean H, independently from each other mean C₁-C₁₄ alkyl, C₂-C₁₄ alkenyl, aryl as defined in R21, C₁-C₄ alkylaryl, heteroaryl as defined in R2, C₁-C₄ alkylheteroaryl, C₃-C₈ cycloalkyl, C₁-C₄ alkylcycloalkyl, heterocycloalkyl namely cycloalkyl groups, wherein up to two CH₂ groups may be substituted by oxygen, sulfur or nitrogen atoms, and another CH₂ group may be substituted by a carbonyl function, C₁-C₄ alkylheterocycloalkyl, CₘH₂ₘ₊ₒ₋ₚYₚ (with m = 1 to 6, for o = 1, p = 1 to 2m+o; for m = 2 to 6, o = -1, p = 1 to 2m+o; for m = 4 to 6, o = -2, p = 1 to 2m+o; Y = independently selected from the group consisting of halogen, OH, OR21, NH₂, NHR21, NR21R22, SH, SR21), or R5 and R6, together with X₁-C-C-X₂, form a ring with 5, 6, or 7 members,
R4, R7, R8 independently from each other mean H, C₁-C₆ alkyl, CO-R41,
R41 independently from R21 has the same meanings as R21,
Y3 means O, S, preferably O,
as well their stereoisomers, tautomers, and their physiologically tolerable salts or inclusion compounds.

4. The compounds according to one of the claims 1 to 3 in the form of their inclusion compounds with cyclodextrin, particularly alpha cyclodextrin.

5. Drugs containing compounds according to one of the claims 1 to 4, as well as the usual carrier and adjuvants.

6. Drugs according to claim 5 in combination with further agents for tumor treatment.

7. The use of compounds according to one of the claims 1 to 4 for preparation of drugs for treatment of tumors.

8. The use of compounds according to one of the claims 1 to 4 for preparation of drugs for treatment of parasites.

9. The use of compounds according to one of the claims 1 to 4 for preparation of drugs for immunosuppression.

10. The use of compounds according to one of the claims 1 to 4 for preparation of drugs for treatment of atopic dermatitis.

## Revendications

1. Composés selon la formule générale Ia ou Ib : 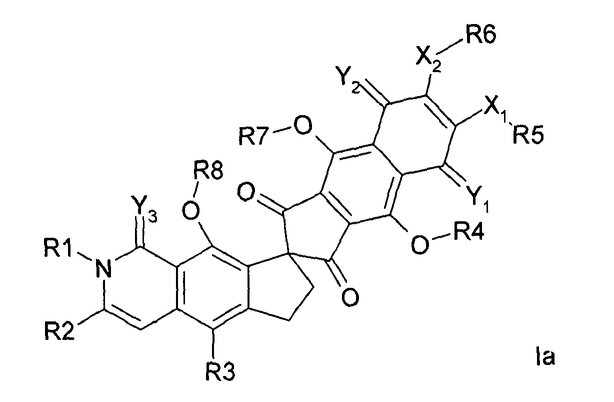 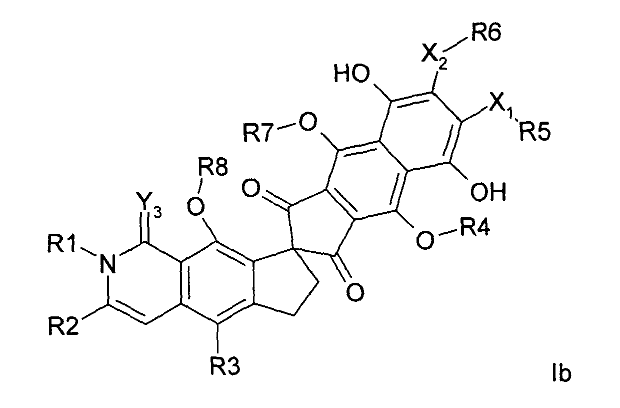 dans lesquelles, respectivement
R1 représente H, alkyle en C₁-C₆, cycloalkyle en C₃-C₈, alkyle en C₁-C₄-cycloalkyle,
R2 représente alkyle en C₁-C₁₄, alcényle en C₂-C₁₄, alkyle en C1-C4-aryle, hétéroaryle et ce des cycles aromatiques avec jusqu'à trois cycles, et jusqu'à trois mêmes ou différents atomes d'hétéro N, S, O lesquels au moins un cycle est aromatique, et lesquels avec jusqu'à trois fois substitué, où les restes représentent indépendamment les uns des autres, alkyle en C1-C6, OH, N02, CN, CF3, OR11, SH, SR11, alkyle en C₁-C₆ hydroxy, alkyle en C₁-C₆-OR11, COOH, COOR11, NH₂, HHR11, NR11R12, halogène, où les restes R11, R12 représentent indépendamment l'un de l'autre, alkyle en C₁-C₁₀, cycloalkyle en C₃-C₈, alkyle en C₁-C₄-cycloalkyle;
alkyle en C₁-C₄-hétéroaryle, hétérocycloalkyle et ce groupes de cycloalkyle où jusqu'à deux groupes de CH₂ peuvent être substitué par atomes d'oxygène, soufre, azote et un outre groupe CH₂ peut être substitué par d'un fonction de carbonyle, alkyle en C₁-C₄-hétérocycloalkyle, CₘH₂ₘ₊ₒ₋ₚYₚ (avec m = 1 à 6, pour o = 1, p = 1 à 2m+o ; pour m = 2 à 6, o = -1, p = 1 à 2m+o ; pour m = 4 à 6, o = -2, p = 1 à 2m+o ; Y = choisi indépendamment l'un de l'autre dans le groupe constitué par halogène, OH, OR21, NH₂, NHR21, NR21R22, SH, SR21), CH₂NHCOR21, CH₂NHCSR21, CH₂S(O)nR21 avec n = 0, 1, 2, CH₂SCOR21, CH₂OSO₂-R21, CHO, CH=NOH, CH(OH)R21,-CH=NOR21, -CH=NOCOR21,-CH=NOCH2CONR21R22,-CH=NOCH(CH₃)CONR21 R22,-CH=NOC(CH3)2CONR21 R22, -CH=N NHCO-R23, -CH=N-NHCO-CH₂NHCOR21, -CH=N-O-CH2NHCOR21, - CH=N-NHCS-R23,-CH=CR24R25 (trans ou cis), COOH, COOR21, CONR21R22, -CH=NR21, -CH=N-NR21R22, 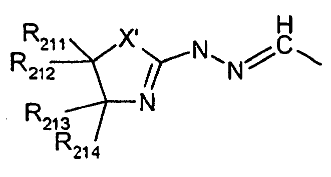 (avec X' = NR215, 0, S et R211, R212, R213, R214, R215 qui représentent indépendamment les uns des autres H ou alkyle en C₁-C₆), -CH=N-NHSO₂-aryle, -CH=N-NHSO₂-hétéroaryle,
R21, R22 représentent indépendamment l'un de l'autre alkyle en C₁-C₁₄, alcanoyle en C₁-C₁₄, alkylhydroxy en C₁-C₆, alkylamino en C₁-C₆, alkylamino en C₁-C₆-alkyle en C₁-C₆, alkylamino en C₁-C₆-di-alkyle en C₁-C₆, cycloalkyle en C₃-C₈, alkyle en C₁-C₄-cycloalkyle, hétérocycloalkyle, alkyle en C1-C4-hétérocycloalkyle, aryle et ce des cycles aromatiques avec jusqu'à trois cycles, lesquels au moins un cycle est aromatique, et lesquels avec jusqu'à trois fois substitué, préféré une fois substitué, où les restes représentent indépendamment les uns des autres, alkyle en C₁-C₆, OH, NO₂, CN, CF₃, OR11, SH, SR11, alkyle en C₁-C₆ hydroxy, alkyle en C₁-C₆ -OR11, COOH, COOR11, NH₂, HHR11, NR11R12, halogène, où les restes R11, R12 représentent indépendamment l' un de l'autre, alkyle en C₁-C₁₀, cycloalkyle en C₃-C₈, alkyle en C₁-C₄-cycloalkyle;
aryloyle, alkyle en C₁-C₄-aryle, hétéroaryle que défini dans R2, hétéroaryloyle, alkyle en C₁-C₄-hétéroaryle, cycloalcanoyle, alcanoyle en C₁-C₄-cycloalkyle, hétérocycloalcanoyle, alcanoyle en C₁-C₄-hétérocycloalkyle, alcanoyle en C₁-C₄-aryle, alcanoyle en C₁-C₄-hétéroaryle, résidus de mono- et disaccharides, qui sont reliés par le biais d'un atome de C qui porterait un groupe OH dans le sucre, où les sucres sont choisis indépendamment les uns des autres dans le groupe constitué de l'acide glucuronique et ses stéréoisomères dans tous les atomes de C optiques, les aldopentoses, les aldohexoses, y compris leurs composés désoxy,
R23 a, indépendamment de R21, les mêmes significations que R21 ou représente des sels de CH₂-pyridinium, des sels de CH₂-tri-C₁-C₆-alkylammonium,
R24 a, indépendamment de R21, les mêmes significations que R21 ou représente H, CN, COCH₃, COOH, COOR21, CONR21R22, NH₂, NHCOR21,
R25 a, indépendamment de R21, les mêmes significations que R21 ou représente H, CN, COCH₃, COOH, COOR21, CONR21R22, NH₂, NHCOR21,
R24, R25 représentent conjointement cycloalkyle en C₄-C₈,
R3 représente H, F, Cl, Br, I, OH, OR31, NO₂, NH₂, NHR31, NR31R32, NHCHO, NHCOR31, NHCOCF3, CH₃₋ₘhalₘ (with hal = Cl, F, especially F, and m = 1, 2,3),OCOR31,
R31, R32 représentent indépendamment les uns des autres H, alkyle en C₁-C₆,
R5, R6 représentent indépendamment les uns des autres H, alkyle en C1-C14, alcényle en C2-C14, aryle que défini dans R21, alkyle en C1-C4-hétéroaryle, cycloalkyle en C3-C8, alkyle en C1-C4-cycloalkyle, hétérocycloalkyle que défini dans R2, alkyle en C1-C4-hétérocycloalkyle, CmH2m+o-pYp (avec m = 1 à 6, pour o = 1, p = 1 à 2m+o ; pour m = 2 à 6, o = -1, p = 1 à 2m+o ; pour m = 4 à 6, o = -2, p = 1 à 2m+o ; Y = choisi indépendamment l'un de l'autre dans le groupe constitué par halogène, OH, OR21, NH2, NHR21, NR21R22, SH, SR21), ou R5, R6 forment conjointement avec X1-C-C-X2 un cycle à 5, 6 ou 7 membres,
R4, R7, R8 représentent indépendamment les uns des autres H, alkyle en C₁-C₆, CO-R41
R41 a, indépendamment de R21, les mêmes significations que R21
X1 représente O, S, NH, alkyle en N-C₁-C₈, N- cycloalkyle,
X2 représente O, S, NH, alkyle en N-C₁-C₈, N- cycloalkyle,
Y1 représente O, S, N-R9, où R9 a, indépendamment de R5, les mêmes significations que R5,
Y2 représente O, S, N-R10, où R10 a, indépendamment de R5, les mêmes significations que R5,
et, si Y1 ou Y2 représente N-R9 ou N-R10, X2-R6 peut représenter H,
Y3 représente O, S, NH,
leurs stéréoisomères, tautomères et leurs sels physiologiquement acceptables ou composés d'inclusion.

2. Composés selon la revendication 1, dans lesquels la formule Ia ou Ib prend la stéréochimie de la formule IIa ou IIb

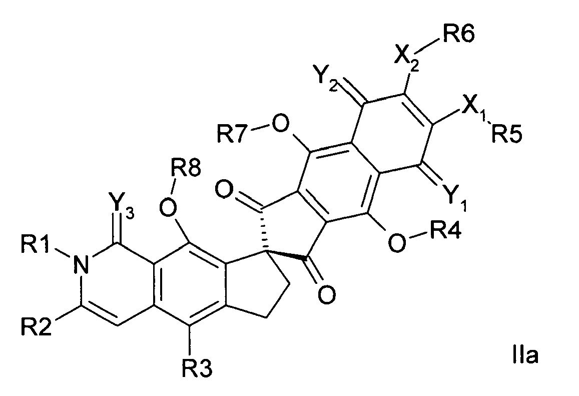

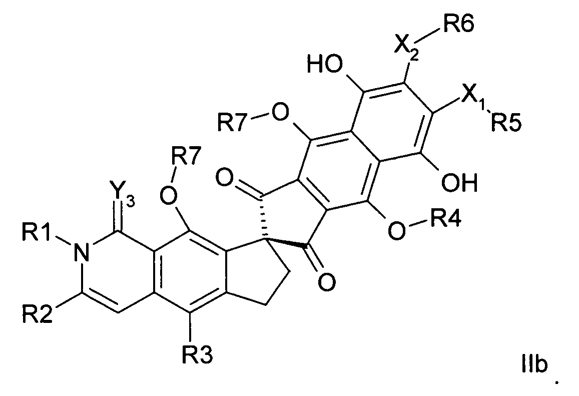

3. Composés selon l'une des revendications 1 à 2, dans lesquels
R1 représente H, alkyle en C₁-C₅, cycloalkyle en C₃-C₈,
R2 représente alkyle en C₁-C₅, alkyle en C₁-C₄-aryle, alcényle en C₂-C₅, hétéroaryle et ce des cycles aromatiques avec jusqu'à trois cycles, et jusqu'à trois mêmes ou différents atomes d'hétéro N, S, O lesquels au moins un cycle est aromatique, et lesquels avec jusqu'à trois fois substitué, où les restes représentent indépendamment les uns des autres, alkyle en C₁-C₆, OH, NO₂, CN, CF₃, OR11, SH, SR11, alkyle en C₁-C₆ hydroxy, alkyle en C₁-C₆ -OR11, COOH, COOR11, NH₂, HHR11, NR11R12, halogène, où les restes R11, R12 représentent indépendamment l' un de l'autre, alkyle en C₁-C₁₀, cycloalkyle en C₃-C₈, alkyle en C₁-C₄-cycloalkyle;
alkyle en C₁-C₄-hétéroaryle, CHF₂, CF₃, chaîne latérale de polyole notamment CHOH-CHOH-CHOH-CHOH-CH₃, CHOH-CHOH-CH=CH-CH₃, CH=CH-CHOH-CHOH-CH₃, CH₂Y (Y=F, Cl, Br, I), CH₂NH₂, CH₂NR21NR22, CH₂NHCOR23, CH₂NHCSR23 CH₂SH, CH₂S(O)nR21 avec n = 0, 1, 2, CH₂SCOR21, en particulier CH₂OH, CH₂OR21, CH₂OSO₂-R21, en particulier CHO, CH(OR21)₂, CH(SR21)₂, CN, CH=NOH, CH=NOR21, CH=NOCOR21, CH=N-NHCO-R23, CH=CR24, R25 (trans ou cis), en particulier COOH (notamment ses sels physiologiquement acceptables), COOR21, CONR21R22, -CH=NR21, -CH=N-NR21R22, 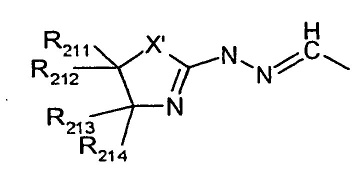 (avec X' = NR215, O, S et R211, R212, R213, R214, R215 qui représentent indépendamment les uns des autres H ou alkyle en C₁-C₆), - CH=N-NHSO₂-aryle, -CH=N-NHSO₂-hétéroaryle, CH=N-NHCO-R23,
R21, R22 représentent indépendamment l'un de l'autre alkyle en C₁-C₆, cycloalkyle en C₃-C₈, aryle et ce des cycles aromatiques avec jusqu'à trois cycles, lesquels au moins un cycle est aromatique, et lesquels avec jusqu'à trois fois substitué, préféré une fois substitué, où les restes représentent indépendamment les uns des autres, alkyle en C1-C6, OH, N02, CN, CF3, OR11, SH, SR11, alkyle en C₁-C₆ hydroxy, alkyle en C₁-C₆ -OR11, COOH, COOR11, NH₂, HHR11, NR11R12, halogène, où les restes R11, R12 représentent indépendamment l' un de l'autre, alkyle en C₁-C₁₀, cycloalkyle en C₃-C₈, alkyle en C₁-C₄-cycloalkyle;
alkyle en C₁-C₄-aryle, hétéroaryle que défini dans R2, alkyle en C₁-C₄-hétéroaryle,
R23 a, indépendamment de R21, les mêmes significations que R21 ou représente des sels de CH₂-pyridinium, des sels de CH₂-tri-C₁-C₆-alkylammonium,
R24 a, indépendamment de R21, les mêmes significations que R21 ou représente H, CN, COCH₃, COOH, COOR21, CONR21R22, NH₂, NHCOR21,
R25 a, indépendamment de R21, les mêmes significations que R21 ou représente H, CN, COCH₃, COOH, COOR21, CONR21R22, NH₂, NHCOR21,
R24, R25 représentent conjointement cycloalkyle en C₄-C₈,
R3 représente F, Cl, Br, I, NO₂, NH₂, NHCOR31,
R31 représente indépendamment l'un de l'autre alkyle en C₁-C₆,
R5, R6 représentent H, indépendamment les uns des autres, alkyle en C₁-C₁₄, alcényle en C₂-C₁₄, aryle que défini dans R21, alkyle en C₁-C₄-hétéroaryle, cycloalkyle en C₃-C₈, alkyle en C1-C4-cycloalkyle, hétérocycloalkyle et ce groupes de cycloalkyle où jusqu'à deux groupes de CH₂ peuvent être substitué par atomes d'oxygène, soufre, azote et un outre groupe CH₂ peut être substitué par d'un fonction de carbonyle;
alkyle en C₁-C₄-hétérocycloalkyle, CₘH₂ₘ₊ₒ₋ₚYₚ (avec m = 1 à 6, pour o = 1, p = 1 à 2m+o ; pour m=2 à 6, o=-1,p = 1 à 2m+o ; pour m = 4 à 6, o = -2, p = 1 à 2m+o ; Y = choisi indépendamment l'un de l'autre dans le groupe constitué par halogène, OH, OR21, NH₂, NHR21, NR21R22, SH, SR21), ou R5, R6 forment conjointement avec X1-C-C-X2 un cycle à 5, 6 ou 7 membres,
R4, R7, R8 représentent indépendamment les uns des autres H, alkyle en C₁-C₆, CO-R41
R41 a, indépendamment de R21, les mêmes significations que R21
X représente O, S, NH, N-R8,
Y représente O, S, NH.

4. Composés selon l'une des revendications 1 à 3 sous la forme de composés d'inclusion avec de la cyclodextrine, notamment l'alpha-cyclodextrine.

5. Médicament contenant des composés selon l'une des revendications 1 à 4 en plus des véhicules et auxiliaires usuels.

6. Médicament selon la revendication 5 en combinaison avec d'autres principes actifs pour le traitement des tumeurs.

7. Utilisation des composés selon l'une des revendications 1 à 4 pour la préparation de médicaments pour le traitement des tumeurs.

8. Utilisation des composés selon l'une des revendications 1 à 4 pour la préparation de médicaments pour le traitement des parasites.

9. Utilisation des composés selon l'une des revendications 1 à 4 pour la préparation de médicaments pour l'immunosuppression.

10. Utilisation des composés selon l'une des revendications 1 à 4 pour la préparation de médicaments pour le traitement de la névrodermite.
